# EUROPÄISCHE PATENTSCHRIFT

(11) **EP 1 141 378 B1**
(45) Veröffentlichungstag und Bekanntmachung des Hinweises auf die Patenterteilung: **08.03.2006**
(21) Anmeldenummer: 99968374.1
(22) Anmeldetag: 22.12.1999
(51) Int. Cl.: C12Q 1/00

(54) **AFFINITÄTSSENSOR FÜR DEN NACHWEIS SPEZIFISCHER MOLEKULARER BINDUNGSEREIGNISSE UND DESSEN VERWENDUNG**
AFFINITY SENSOR FOR DETECTING SPECIFIC MOLECULAR BINDING EVENTS AND USE THEREOF
DETECTEUR D'AFFINITE POUR DECELER DES EVENEMENTS DE LIAISONS MOLECULAIRES SPECIFIQUES ET SON UTILISATION

(30) Priorität: 23.12.1998 DE 19860547
(43) Veröffentlichungstag der Anmeldung: 10.10.2001
(73) Patentinhaber: Genetrix B.V., 9751 SJ Haren (NL)
(72) Erfinder: FRITZSCHE, Wolfgang, D-07743 Jena (DE); CZAKI, Andrea, D-07774 Camburg (DE); KÖHLER, Johann, Michael, D-07751 Golmsdorf (DE); OOSTING, Louis, NL-9722 RP Groningen (NL); SCHUT, Frederik, NL-9728 VM Groningen (NL); TAN, Paris, Som, Tjwan, NL-9751 SJ Haren (NL); WIEGAND, Antje, D-07743 Jena (DE)
(74) Vertreter: Prins, Adrianus Willem
(86) Internationale Anmeldenummer: PCT/EP1999/010334
(87) Internationale Veröffentlichungsnummer: WO 2000/039325

(56) Entgegenhaltungen:
- WO-A-88/08528
- WO-A-97/41425
- DE-A- 19 517 789
- US-A- 5 457 396
- US-A- 5 494 831

## Beschreibung

Die Erfindung betrifft einen Affinitätssensor für den Nachweis spezifischer molekularer Bindungsereignisse, wie er insbesondere auf dem molekularbiologischen Gebiet, bspw. der medizinischen Diagnostik, der Biosensor- oder DNA-Microarray-Technologie, eingesetzt wird, und dessen Verwendung.

Biosensoren sind Festphasenmeßapparaturen, die aus mindestens einem biologischen Rezeptor, einem Wandler und einer nachgeschalteten Elektronik bestehen.
Bei dem Rezeptor finden biologisch aktive Reagenzien, wie bspw. Antikörper, Anwendung, um eine bestimmte Substanz, wie bspw. Antigene, zu erkennen. Die Transduktion der Erkennungsereignisse in detektierbare Signale erfolgt durch den Wandler, bspw. mit elektrochemischen, optischen, piezoelektrischen oder kalorimetrischen Methoden. Dabei kann die Kopplung der Erkennungsereignisse an den Wandler auf indirektem oder direktem Wege erfolgen. Im ersten Fall modulieren die Erkennungsereignisse einen Prozeß, der von dem Wandler detektiert wird. Im zweiten Fall werden die Erkennungsereignisse selbst durch den Wandler aufgezeichnet. Der Wandler steht mit einer Elektronikeinheit, bspw. einem Mikroprozessor mit nachgeschalteten Modulen, zur Signalerfassung und Auswertung in Verbindung.
Die Anwendungsmöglichkeiten dieser, auf Grundlage der molekularen Erkennung arbeitenden Biosensoren ist vielfältig. Sie liegen u.a. auf dem Gebiet des Nachweises und der Konzentrationsbestimmung von Biomolekülen, der kinetischen und der Gleichgewichts- Analyse von biochemischen Reaktionen, der Überwachung von Fermentationsprozessen, der Charakterisierung von Rezeptor-Zell-Wechselwirkung, der klinischen Analyse und der Zell-Detektion.
Die Detektion der Aüwesenheit bioaktiver Moleküle erfolgt im Fall der Nukleinsäuren bspw. durch Hybridisierung mit spezifischen und markierten Nukleinsäuresonden. Die Markierung der Sonden erfolgt durch den enzymatischen Einbau von Nukleotiden, die Radioisotope, wie bspw. Tritium, Schwefel-35 oder Phosphor-32, nichtradioaktive Moleküle, wie bspw. Digoxigenin oder Biotin bzw. nichtradioaktive, fluoreszierende Moleküle, wie bspw. Fluoresceinisothiocyanat oder 7-Amino-4-methylcumarin-3-acetat oder metallische Partikel, wie bspw. Gold, tragen (Nicholl, D., S., T., 1995: Genetische Methoden, Spektrum Akademischer Verlag Heidelberg, S.24-27).
Die Detektion der Anwesenheit bioaktiver Moleküle erfolgt im Fall von Antigenen, z.B. Peptiden oder Proteinen, mit spezifischen und markierten Antikörpern. Die Markierung der Antikörper erfolgt durch Ankoppeln von Radioisotopen, bspw. lod-125 oder Tritium, an Tyrosin- bzw. Histidinreste, durch nichtradioaktive Enzyme, bspw. alkalische Phosphatase oder Peroxidase, wobei die enzymatische Aktivität, bspw. durch die Umsetzung eines farblosen in ein farbiges Produkt, gemessen wird, durch nichtradioaktive Enzyme, bspw. Hämatin, das die chemilumineszente Reaktion von Wasserstoffperoxid und Luminol bewirkt, durch nichtradioaktive Enzyme, bspw. Luciferase, die Bioluminiszenz mittels phosphorilierten Luciferin bewirkt, oder durch metallische Partikel, bspw. Gold (Liddell, E. und Weeks, I: 1996: Antikörpertechniken, Spektrum Akademischer Verlag Heidelberg, S.87-107).
Die Signale der verwendeten verschiedenen Markermoleküle werden durch radio- oder elektrochemische, optische, piezoelektrische oder kalorimetrische Verfahren zur Darstellung von molekularen Erkennungsereignissen ausgewertet. Die Größe der Einzelsignalaussendenden Markermoleküle liegt dabei im Nanometerbereich.
Derzeit am meisten angewendet werden die optischen und die elektrochemischen Verfahren zur Darstellung von molekularen Bindungsereignissen.
Das Problem der verschiedenen optischen Verfahren ist, daß die Sensitivität und die räumliche Auflösung der von den einzelnen Markermolekülen ausgesendeten Signale für viele Anwendungen zu gering ist, eine Bindung zwischen zwei Gliedern eines spezifischen molekularen Bindungspaares nicht nachgewiesen werden kann und daß oft ein unspezifiscber Hintergrund die Signale überlagert. Diese Probleme der bildgebenden Verfahren können nur zum Teil durch eine experimentelle Verstärkung des Signals oder durch computergestützte statistische Bildanalyseverfahren bereinigt werden.
Eine technische Begrenzung der derzeitigen Automatisierung der Bildanalyse auf der Grundlage der Chip-Technologie liegt im Auslesen der verschiedenen Microarray-Spots. Die meisten verfügbaren Technologien beruhen auf der Detektion von fluoreszenzmarkierten Bindungspaaren, die in einer spezifischen Art auf der Chip-Oberfläche festgehalten sind, wobei die Fluoreszenzdetektion durch optisches Auslesen der reaktiven Zentren des Microarrays ausgeführt wird. Der Gebrauch von fluoreszierenden oder chemilumeniszierenden Proben findet dabei wie in den zuvor beschriebenen klassischen Methoden Anwendung und wird mit CCD-Imaging kombiniert (Eggers, M. et al., 1996: Professional Program Proceedings. Electro '96. IEEE, New York, NY, USA, 364pp.; Heller, M.J., 1996: IEEE-Engineering-in-Medicineand-Biology-Magazine 15: 100-104), wobei auch hier die genannten Probleme der klassischen Bildanalyse auftreten und eine Bindung zwischen zwei Gliedern eines spezifischen molekularen Bindungspaares nicht nachgewiesen werden kann.
Die Detektion der Anwesenheit bioaktiver Moleküle kann neben den sehr häufig verwendeten optischen Verfahren auch auf elektrochemischem Wege durch verschiedene Verfahren erfolgen.
Bekannt ist die Möglichkeit der Messung von Redoxpotential-Änderungen bei Biomolekülen, die mit spezifischen Bindungsereignissen, bspw. an Enzymen, einhergehen. Dabei werden die Redoxpotential-Änderungen mittels einer mit Biomolekülen versehenen Einzelelektrode und einer Referenz-Elektrode gemessen (Heller, A., 1992: Electrical connection of enzyme redox centers to electrodes, J. Phys. Chem. 96: 3579-3587).
Der Nachteil dieser Methode ist, daß nur ein einzelnes elektronisches Ereignis für ein biomolekulares Bindungsereignis eintritt, wobei die Änderung des Redox-Zustandes, die bewirkt wird, nur kurzzeitig ist und somit die Detektion jedes individuellen Bindungsereignisses blitzartig erfolgen müßte; dies ist nicht möglich. Das erhaltene Signal ist lediglich kumulativ, so daß seltene Bindungsereignisse mit dieser Technologie nicht detektiert werden können.
Eine weitere Möglichkeit zur Detektion der Anwesenheit bioaktiver Moleküle auf dem elektrischen Weg stellen die Biosensoren in Form von speziellen Meßelektroden dar. Diese speziellen Meßelektroden bestehen im allgemeinen aus einer (Strept-)Avidin-beschichteten Elektrode, wobei das (Strept-)Avidin die Eigenschaft besitzt, Biotinmoleküle spezifisch zu binden. Dadurch ist es möglich, Peptide, Oligonukleotide, Oligo- und Polysacchsaride sowie Lipide, die mit Biotin oder Biotinderivaten markiert sind, zu detektieren, bzw. diese an die (Strept-)Avidin-Schicht als Liganden zu koppeln, wobei in diesem Fall die Biotinmoleküle die Kopplungselemente darstellen. Im allgemeinen können mit diesen Biosensoren Antikörper / Antigen-, Antikörper / Hapten-, Saccharid / Lectin-, Protein / Nukleinsäure- und Nukleinsäure/Nukleinsäure- Bindungspaare detektiert werden. Die Detektion der an der speziellen Meßelektrode eintretenden biochemischen Ereignisse erfolgt in ähnlicher Form wie bei der zuvor besclzicbenen Technologie, die auf Redoxsytemen beruht, durch Messungen der Potential-Änderungen einer Einzelelektrode im Vergleich zu einer Referenz-Elektrode (Davis, et al., 1995: Elements of biosensor construction. Enzyme Mirob. Technol. 17: 130-1035).
Ein wesentlicher Nachteil dieser konventionellen Biosensortechnologie ist dabei die geringe Sensibilität der über die Meßelektroden erzielten Messungen, der nicht dadurch behoben werden kann, daß Liganden in unendlich großer Dichte auf die Meßelektrode, bspw. durch die Verwendung einer Dextranschicht, gebunden werden. Durch das zusätzliche Aufbringen, bspw. einer Dextranschicht, wird zwar durch die rämliche Ligandenanordnung eine Steigerung auf die bis zu sechsfache Ligandenkonzentration gegenüber der Ligandeneinfachschicht auf den Elektroden erreicht, ein Nachweis von seltenen Bindungsereignissen oder gar von einer Bindung zwischen zwei Gliedern eines spezifischen molekularen Bindungspaares ist jedoch nicht möglich.
Bekannt sind auch die Möglichkeit der Verankerung von spezifischen Antikörpern auf einem Halbleitertor eines Feldeffekttransistors, wobei durch die selektive Bindung von Antigenen an die spezielle Antikörperschicht eine Veränderung in der Ladungsverteilung und damit in der Schaltung des Feldeffekttransistors erfolgt, die Möglichkeit der Immobilisierung von spezifischen Antikörpern auf der Oberfläche einer optischen Faser, wobei durch die selektive Bindung von Antigenen an die spezielle Antikörperschicht an der Schnittstelle von Faseroptik und Flüssigkeit meßbare optische Phänomene, wie bspw. interferierende Wellen und Oberflächenplasmonen, auftreten sowie die Methode der Oberflächenplasmonenresonanz, bei der bei einem definierten Einfallswinkel des Lichtes der Refraktionsindex eines Mediums an einem metallbeschichteten, mit spezifischen Antikörpern versehenen Glaskörper durch die selektive Kopplung von Antigenen meßbar verändert wird (Liddell, E. und Weeks, I: 1996: Antikörpertechniken, Spektrum Akademischer Verlag Heidelberg, S.156-158).
Der Nachteil dieser Methoden ist, daß seltene Bindungsereignisse mit diesen Technologien nicht detektiert werden können.

Obgleich der biochemische Prozeß der Bindungspaarbildung bei Biosensoren, z.B. die Hybridisierung von zwei Nukleotidsträngen oder die Bindung von Antikörper an Antigen, selbst sehr schnell, d.h. im Sekundenbereich, verläuft, Biochips mit Bindemolekülen, z.B. spezifischen Oligonukleotiden (US 5,445,934) oder spezifischen Proteinen (US 5,077,210), bestückt werden können, so daß eine Chip-Technologie möglich ist (Osborne, J.C., 1994: Genosensors. Conference Record of WESCON/94. Idea/Microelectronics. IEEE, New York, NY, USA: 434pp.; Eggers, M.D. et al., 1993: Genosensors, microfabricated devices for automated DNA sequence analysis. Proc. SPIE-Int. Soc. Opt. Eng. 1998), durch die innerhalb von Minuten die Gegenwart von bestimmten Biomolekülen, bspw. Genen durch spezifische Oligonukleotid-Sonden oder Antigene durch spezifische Antikörper detektierbar ist und große Perspektiven auf dem Gebiet der Biologie oder Medizin, vor allem bei genetischen Untersuchungen, aufzeigt werden (Chee, M. et al., 1996: Accessing genetic information with high-density DNA arrays. Science 274: 610-614), gibt es zur Zeit nur wenige Methoden, mit denen Bindungen zwischen Molekülen bei niedrigen Konzentrationen oder gar bei einzelnen Molekülpaaren schnell festgestellt werden können (Lemieux, B., et al., 1998: Overview of DNA chip technology. Molecular Breeding 4: 277-289).
Ein vielversprechender Ansatz für die Detektion von Bindungsereignissen zwischen Nukleinsäure-Bindungspaaren besteht derzeit im Ausnutzen von dielektrischen Relaxationsfrequenzen der DNA zur Unterscheidung zwischen hybridisierten und nicht hybridisierten Proben (Beattie et al. 1993. Clin. Chem. 39: 719-722). Die Detektion dieser Frequenzunterschiede erfordert jedoch bisher sehr teure Ausrüstungen und ist darüber hinaus noch weit davon entfernt, routinemäßig angewendet zu werden.
Darüber hinaus ist ein weiterer Weg hybridisierte und nicht hybridisierte Proben elektronisch zu unterscheiden bekannt, der darin besteht, die Geschwindigkeit der Elektronenbewegung entlang der DNA-Stränge zu bestimmen (US 5,780,234). Diese Bestimmung basiert darauf, daß die Anordnung der Pi-Elektronenorbitale in doppelsträngiger DNA die Elektronen dazu veranlaßt, sich in doppelsträngiger, also hybridisierter DNA schneller zu bewegen als in einzelsträngiger DNA (Lipkin et al., 1995: Identifying DNA by the speed of electrons. Science News 147: 117pp.). Die Zielprobe muß dabei, um diese Elektronenbewegung zu bestimmen, exakt zwischen zwei Molekülen positioniert werden, von denen das eine chemisch so modifiziert ist, daß es als Elektronendonator dient, und das andere so, daß es als Elektronenakzeptor fungiert, so daß ein Elektronenfluß über Elektroden meßbar wird.
Diese aufwendige Methode besitzt den Nachteil, daß sie die Anwendung auf die Detektion von Einzelstrang-Nukleinsäurefragmenten von definierter Länge limitiert und für weitere Biomoleküle nicht geeignet ist.

Eine Methoden zur elektrischen Detektion von Partikeln ist darüber hinaus durch Bezryadin, A., Dekker, C., und Schmid, G., 1997: Electrostatic trapping of single conducting nanoparticles between nanoelektrodes. Applied Physics Letters 71: 1273-1275, bekannt, wobei Nanopartikel in einen Elektrodenspalt gebunden werden, indem eine Spannung an die Elektroden angelegt wird und das Einfangen des Partikels anhand des fließenden Stromes detektiert wird. Im Gegensatz zu Bindungsereignissen von Biomolekülpaaren erfolgt dabei keine spezifische biochemische Bindung des Nanopartikels, sondern der Partikel wird durch das elektrische Feld an den Elektrodenspalt gebunden.
Aus einer Arbeit von Braun, E., Eichen, Y., Sivan, U. und Ben-Yoseph, G., 1998: DNA templated assembly and electrode attachment of a conducting silver wire. Nature 391: 775-778, ist weiterhin bekannt, daß DNA-Moleküle zwischen zwei mikrostrukturierte Elektroden spannbar sind und diese Moleküle erst nach ihrer Beschichtung mit Silber eine elektrische Leitfähigkeit zeigen, die jedoch in keinem Zusammenhang mit spezifischen biochemischen Bindungsereignissen von Biomolekülpaaren steht.
Durch Alivisatos, A., P., Johnsson, K., P., Peng, X., Wilson, T., E., Loweth, C., J., Bruchez Jr., M., P. und Schulz, P., G., 1996: Organization of nanocrystal molecules using DNA. Nature 382: 609-611, wurden Komplexe aus kurzen, einzelsträngigen DNA-Molekülen und ihren komplementären, einzelsträngigen, mit Goldpartikel markierten DNA-Molekülen in Lösung erzeugt und für die elektronenmikroskopische Charakterisierung auf ein TEM-Grid mit Kohlenstofffilm aufgebracht. Eine elektrische Charakterisierung der Molekülpaarbindung erfolgte dabei nicht.

Der Erfindung liegt die Aufgabe zugrunde, einen Affinitätssensor für den Nachweis spezifischer molekularer Bindungsereignisse zu schaffen, welcher in schneller, empfindlicher, spezifischer, aufwandgeringer und routinemäßiger Weise die Anwesenheit von Molekülen, insbesondere von bioaktiven Molekülen detektiert, sowie spezielle Verwendungsmöglichkeiten für einen derartigen Affinitätssensor anzugeben.

ErfindungsgemäB wird die Aufgabe durch die Merkmale der Ansprüche gelöst. Insbesondere besteht ein Affinitätssensors aus einem Träger, welcher mit beabstandeten Elektroden versehen ist, die einen Bereich erfassen, der mit immobilisierten spezifischen Bindungspartnern versehen ist, welche komplementär zugehörige, elektrisch leitfähige Partikel tragende Bindungspartner spezifisch koppeln, so daß durch die Partikel ein elektrisch leitender Kontakt zwischen den Elektroden bildbar ist und dadurch die Bestimmung der Änderung des elektrischen Widerstandes bei an die Elektroden angelegter Spannung sowie Anwesenheit von einzelnen oder mehreren komplementär zugehörigen, elektrisch leitfähige Partikel tragenden Bindungspartnern detektierbar ist.

Im folgenden wird die Erfindung anhand von Ausfühmngsbeispielen und zugehörigen schematischen Zeichnungen näher erläutert. Es zeigen:
- Fig. 1: einen Affinitätssensor für den Nachweis spezifischer molekularer Bindungsereignisse,
- Fig. 2: eine schematische Darstellung des Affinitätssensor für den Nachweis spezifischer molekularer Bindungsereignisse,
- Fig. 3: einen Querschnitt durch eine Ausführungsmöglichkeit des Affinitätssensors für den Nachweis spezifischer molekularer Bindungsereignisse,
- Fig. 4: eine Ausführung des Affinitätssensors in Form eines Affinitäts-Chips in der Draufsicht,
- Fig. 5: einen Schnitt durch den in Fig. 4 dargestellten Affinitäts-Chips entlang der Ebene A-A.

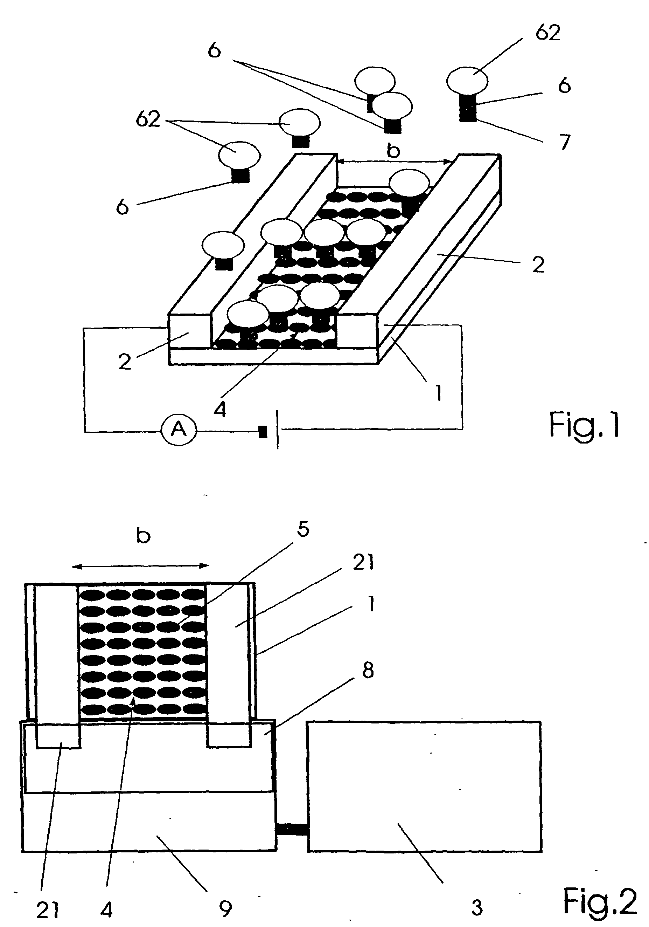

Der in den Figuren 1 und 2 dargestellte Affinitätssensor für den Nachweis spezifischer molekularer Bindungsereignisse besteht aus einem Trägersubstrat 1, das mit Elektroden 2 versehen ist, die einen Bereich 4 umgeben, der immobilisiert spezifische Bindungspartner 5 aufweist. Der Bereich 4 stellt dabei eine Diskontinuität in einem elektrischen Schaltkreis dar, welcher einen Verstärkerschaltkreis 8, der Teil eines Mikrochips 9 sein kann, sowie eine Meß- und Auswerteeinheit umfaßt, wobei die Elektroden 2, die den Bereich 4 begrenzen, im Beispiel dem elektrischen Schaltkreis zugeordnet sind und eine Mindestbreite b des Bereichs 4 bestimmen. Die spezifischen Bindungspartner 5 sind befähigt, komplementär zugehörige Bindungspartner 6 spezifisch direkt oder über weitere spezifische Bindemoleküle 7 zu koppeln, wobei die komplementär zugehörigen Bindungspartner 6 mit elektrisch leitfähigen Partikeln 62 direkt oder über verbindende Moleküle gekoppelt sind. Der Bereich 4 ist durch die Anordnung der Elektroden 2 in seiner Breite und Wirkhöhe so bemessen, daß die Kopplung der immobilisierten spezifischen Bindungspartner 5 mit den komplementär zugehörigen, die leitfähigen Partikel 62 tragenden Bindungspartnern 6 oder den über weitere spezifische Bindemoleküle 7 mit den komplementär zugehörigen, die leitfähigen Partikel 62 tragenden Bindungspartnern 6 möglich ist. Für den Fall, daß die spezifischen Bindungspartner 5 durch Moleküle einer Nukleinsäuresondenspezies realisiert sind, die komplementär zugehörigen, die leitfähigen Partikel 62 tragenden Bindungspartner 6 Nukleinsäuren und die Partikel 62 Nanopartikel einer Größe von 20 nm sind, beträgt die Mindestbreite b des Bereichs 4 25 nm sowie seine Wirkhöhe 20 nm.
Die Kopplung der spezifischen Bindungspartner 5 in den Bereich 4 mit den komplementär zugehörigen, die leitfähigen Partikel 62 tragenden Bindungspartnern 6 bewirkt bei einer an die Elektroden 2 angelegten Spannung (vgl. Fig. 1) die Bewegung von Elektronen über die Elektronentransportbarriere in der Art, daß die elektrisch leitfähigen Partikel 62 den Bereich 4 überbrücken und die Elektronen von Partikel 62 zu Partikel 62 und zu den Elektroden 2 tunneln, so daß eine permanente Änderung des elektrischen Widerstandes über den Bereich 4 zwischen den Elektroden 2 vermittels des nachgeschalteten Verstärkerschaltkreises 8 und der gekoppelten Meß- und Auswerteeinheit 3 meßbar ist.
Anstelle der Messung im Trockenen kann die Messung auch in feuchter Umgebung, insbesondere mit Hilfe einer Gelschicht vorgenommen werden.
Um die durch den komplementär zugehörigen Bindungspartner 6 mit gekoppelten elektrisch leitfähigen Partikeln 62 vermittelte elektrische Leitfähigkeit an dem Bereich 4 zwischen den Elektroden 2 zu verbessern, können bereits bekannte Elektronen-Transfer-Mediatoren oder effektive diffusive Elektronendonatoren und -akzeptoren wie wasserlösliche Ferrocen/Ferricinium, Hydrochinon/Chinon, reduzierbare und oxidierbare Komponenten von organischen Salzen, Cobaltocene, Hexa- und Octacyanide von Molybden, Wolfram und Eisen, bzw. Makrozyklen und Chelatliganden von Übergangsmetallen wie Kobalt, Ruthenium, und Nickel, einschließlich Co(ethylendiamin)3- und Ru(ethylendiamin)3- und Trisbipyridyl- und Hexaminkomplexe von Übergangsmetallenwie Co, Ru, Fe und bzw. organische Moleküle wie 4-4'-bipyridin und 4-mercaptopyridin verwendet werden, die frei in Lösung oder in einem, auf dem Trägersubstrat 1 aufgebrachten Gel oder in einem, auf dem Trägersubstrat 1 aufgebrachten Polymer vorliegen. Eine bekannte Matriximmobilisierung durch Gel besitzt im Fall der Anwendung von Nukleinsäuren als spezifische Bindungspartner 5 wegen des dreidimensionalen Aufbaus des Polymers den Vorteil, daß eine größere Anzahl Einfangliganden auf dem kleinen Oberflächenausschnitt des Bereichs 4 immobilisiert wird. Durch die Verwendung eines hochporösen Hydrogels wird bspw. die Hybridisierungrate der Nukleinsäuren, die spezifische Bindungspartner 5 und komplementär zugehörige, die elektrisch leitfähigen Partikel 62 tragenden Bindungspartner 6 darstellen, erhöht und liegt in Bereichen wie sie für Nukleinsäuren in Lösung bekannt ist.

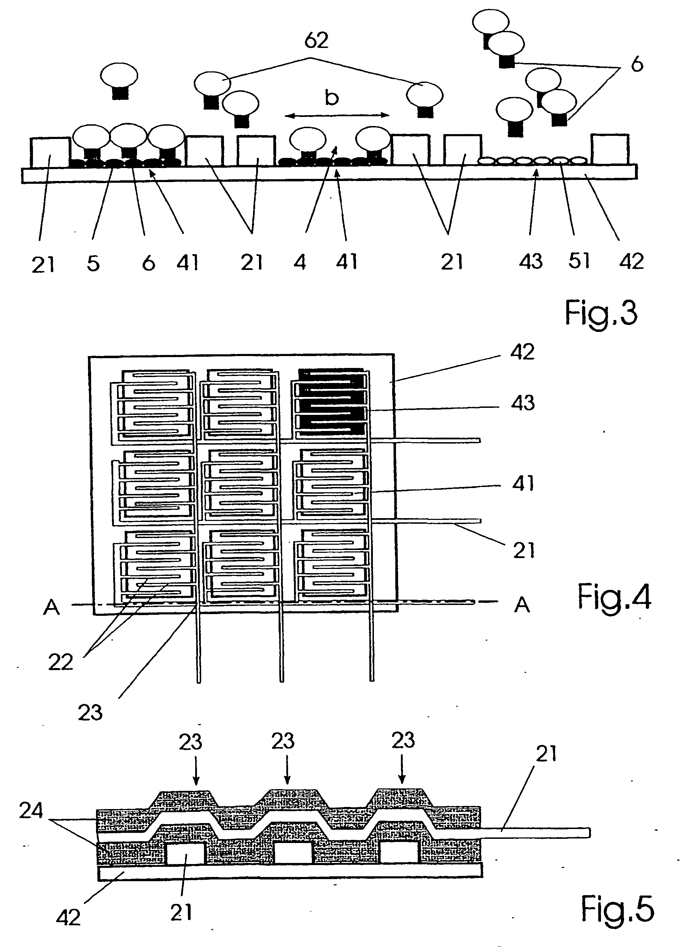

Der in den Figuren 3 und 4 dargestellte Affinitätssensor in Form eines Affinitäts-Chips ist dadurch gekennzeichnet, daß die Elektroden 2 als jeweils zwei paarweise angeordnete, eine Affinitätsfläche 41 erfassende Mikroelektroden 21 ausgebildet sind, so daß eine Matrix von Affinitätsflächen 41 vorliegt, die eine Vielzahl von verschiedenen Kopplungen an den verschiedenen Zwischenrämen 4 zeitgleich elektrisch detektiert.

Die einzelnen Affinitätsflächen 41 sind dabei in einer interdigitalen Elektrodenstruktur auf einer aus bspw. mit einer dielektrischen Oxidschicht versehenen Silizium oder Glas bestehenden Chipfläche 42 ausgebildet. Durch die digital verzweigten, an ihren Schnittstellen 23 durch eine, wie in Fig. 5 dargestellt, zwischengelagerte Isolierschicht 24 voneinander räumlich und elektrisch getrennten Mikroelektroden 21, die bspw. in Form von Kammelektroden 22 gefertigt sein können, werden auf den Affinitätsflächen 41 die Bereiche 4 mit einer Länge im Bereich von 20 µm festgelegt, wobei für den Fall, daß die spezifischen Bindungspartner 5 durch Moleküle einer Nukleinsäuresondenspezies realisiert sind, die komplementär zugehörigen, die leitfähigen Partikel 62 tragenden Bindungspartner 6 Nukleinsäuren und die Partikel 62 Nanopartikel einer Größe von 20 nm sind, die Bereiche 4 eine Wirkhöhe von 100 nm und Breite von 200 nm besitzen, so daß mindestes eine, einen Kontakt zwischen den Elektroden 21 bewirkende Kopplung zwischen den immobilisierten spezifischen Bindungspartnern 5, die in diesem Beispiel Einfangliganden in Form von Nukleinsäuresonden darstellen, und den komplementär zugehörigen, die elektrisch leitfähigen Partikel 62 tragenden Bindungspartnern 6, die in diesem Beispiel Targetmoleküle in Form von Nukleinsäuren darstellen, erfolgt. Die als spezifische Bindungspartner 5 immobilisierten Oligonukleotidsonden werden dabei über eine Aminogruppe am silanisierten Trägersubstrat 1 gebunden, wobei die ezzielte Sondendichte in diesem Beispiel in einer Größenordnung von 10000 Molekülen je µm² liegt. Die komplementär zugehörigen Bindungspartner 6 sind in dem gewählten Beispiel Oligonukleotide, die mit Goldpartikeln markiert sind und die Hybridisierungsbedingungen hängen jeweils von den verwendeten Sonden ab.
In einer bevorzugten Ausführung betragen die Länge der Mikroelektroden (21) 0,1 mm, die Breite b des Bereichs (4) 0,1 µm und seine Wirkhöhe 0,02 µm sowie die Affinitätsfläche (41) zur Chipfläche (42) in einem Verhältnis von 1:10 steht.
Alternativ dazu können die Affinitätsflächen 41 in jeweils voneinander getrennten Sektoren mit verschiedenen immobilisierten spezifischen Bindungspartnern 5. bestückt sein.
Auf dem in den Figuren 3 und 4 dargestellten Affinitäts-Chip sind Affinitftsflächen 41 mit immobilisierten spezifischen Bindungspartnern 5 und Referenzflächen 43 mit immobilisierten inaktiven Bindungspartnern 51 vorgesehen, so daß die Messung des elektrischen Widerstandes zwischen den Mikroelektroden 21, die als Kammelektroden 22 gefertigt sein können, im Vergleich des elektrischen Widerstandes einer Affinitätsfläche 41 zu einer Referenzfläche 43 erfolgt. Dabei können die immobilisierten spezifischen Bindungspartner 5 und die immobilisierten inaktiven Bindungspartnern S auch die Elektroden 21 mit einer, einen Tunneleffekt zulassenden Dicke überdecken, wodurch eine technologisch einfachere Fertigung der Chips ermöglicht wird.
Da die Referenzfläche 43 durch die Besetzung mit inaktiven Bindungspartnern 51 frei von immobilisierten spezifischen Bindungspartnern 5 ist, stellt dieser Raum zwischen den zwei isolierten Mikroelektroden 21 eine elektrische Barriere dar, so daß ein meßbarer Elektronentransfer zwischen ihnen nicht stattfindet.
Die Affinitätsfläche 41, die hingegen immobilisierte spezifische Bindungspartner 5 trägt, bindet über diese durch das Kopplungsereignis die komplementär zugehörigen, elektrisch leitfähige Partikel 62 tragenden Bindungspartner 6, so daß dadurch durch die leitfähigen Partikeln 62 der Ramn der Affmitätsfläche 41 zwischen den Mikroelektroden 21, die in Form der Kammelektroden 22 ausgebildet sind, in eine Vielzahl von nanometerbreiten Spalten geteilt wird. Die so durch die elektrisch leitfähigen Partikel 62 gebildeten Nanospalte führen dazu, daß ein Elektronentransfer zwischen den zwei Kontaktflächen der Mikroelektroden 21 durch Tunneleffekte möglich ist, wodurch die Änderung des Widerstandes über die Vetstärkereinheit 8 mittels einer Meß-und. Auswerteeinheit 3 bei an die Mikroelektroden 21 angelegter Spammung ermöglicht wird. Die angelegte Spammung liegt im Beispiel in der Größenordnung von unter einem Volt.
Alternativ zu der Messung des über der Affinitätsfläche 41 angelegten Potentials durch das Elektrodensystems aus Referenz-, Proben- und Gegenelektrode können auch andere Methoden der elektrischen Detektion, bspw. potentiometrische oder voltametrische Messungen angewendet werden.
Für die Immobilisierung der spezifischen Bindungspartner 5 bzw. die inaktiven Bindungspartner 51, wie z.B. Antikörper oder Nukleotidsonden, werden chemische Standardlinker genutzt, wie z.B. amino-modifizierte Liganden, so daß sie an die silanisierte Chipfläche 42 gebunden sind und die Affinitätsflächen 41 bzw. die Referenzflächen 43 bilden.
Die Markierung der komplementär zugehörigen Bindungspartner 6, wie z.B. der Target-Proteine oder der Target-Nukleinsäure, mit elektrisch leidenden Partikeln 62 wird nach den bekannten Verfahren, so z.B. die Endmarkierung mit markierten Oligonukleotiden durch Verwendung von Ligasen durchgeführt.

Im nachstehenden soll eine detailliertere Beschreibung der Herstellung von Affinitätssensoren nach der Erfindung erfolgen. In einer bevorzugren Ausführung beinhaltet der affinitätssensor mehrere Bereiche 4 (auch als Detektionsbereiche bezeireichnet), die jeweils von mindestens zwei Elektroden 2 erräßt sind. In einer bevorzugten Ausführung is dem Bereich (4) eine Breite b unterhalb von 800 nm gegeben. Diese Detektionsbereiche sind mit spezifischen Bindungsparmern (capture-molecules) 5 versehen, wie DNA-, RNA- oder PNA-Oligonukleotiden, an welche bestimmte zugehörige Bindungspartner (target molecules) 6 in einer spezifischen Weise binden. Die Bindungspartner 5 werden als markierte oder nicht markierte Moleküle definiert, welche ansgewählbar sind zur Bindung des gewünschten Zielmoleküls auf den Bereichen 4 des Affinitätssensors. Dabei sind nicht nur konventionelle (bio)molekulare Bindungspaare als Fang- und Zielmolekül einsetzbar, sondern auch spezifische chemische Bindungspaare, wie sie in der kombinatorischen Chemie bekannt sind, welche im Rahmen der Erfindung einsetzbare Bindungspaare darstellen. Die Ausbildung dieser beschriebenen spezifischen Bindung kann als Primärbindungsereignis aufgefaßt werden. Die Detektion dieser Primärbindung kann in einem Ein- oder Mehrschrittprozeß erfolgen, wobei im jeweils letzten Schritt die spezifische Koimmobilisierung von elektronentransferierendem Material, wie z.B. Goldpartikel 62, erfolgt. Die genannte Koimmobilisierung kann durch spezifische oder unspezifische Arten molekularer Interaktionen geschehen, wie der Hybridisierung von goldmarkierten Sonden auf das gewünschte Zielmolekül oder direkter Markierung des Zielmoleküls mit Elektronentransport-Eigenschaften derart, daß diese Markierung elektronisch detektiert werden kann. Die genannte Koimmobilisierung ist im Prinzip getrennt vom Primärbindungsereignis, ist aber abhängig davon und kann simultan erfolgen. Die Koimmobilisierung oder Anlagerung von elektronentransferierendem Material an der dafür vorgesehenen Oberfläche des Affinitätssensors kann so als indirektes Resultat der Primärbindung erfolgen. Die Detektion dieser Koimmobilisierung erfolgt durch elektronische Messungen der elektrischen Leitfähigkeitsänderung über den Meßbereich, und diese Leitfähigkeitsänderung zeigt die Anwesenheit der Zielmoleküle an. Die primäre Bindung elektronentransferierenden Materials kann benutzt werden, um sekundäre Ablagerungen zu induzieren, welche elektronentransportierend sind. Es liegt im Rahmen vorliegender Erfindung, daß die spezifische Bindung von Zielmolekülen durch einem Mehrschrittprozeß detektiert werden kann, der wenigstens einen Schritt enthält, bei denen elektronentransferierendes Material abgelagert wird, welches eine Verringerung des elektrischen Widerstandes über den Meßbereich bewirkt. Bei dem für die Partikel 62 eingesetzten elektronenleitfähigen Materials können organische oder anorganische Stoffe oder Verbindungen verwendet werden. Diese Leitfähigkeit wird zur Detektion and Markierung der gewünschten Zielmoleküle, d.h. zum Nachweis ihrer Anwesenheit, benutzt.

Im nachfolgenden sollen, ohne die Erfindung darauf zu beschränken, verschiedene Möglichkeiten für Präparationsstufen zur Fertigung eines Affinitätssensors nach vorliegender Erfindung beschrieben werden.
A. Zur Präparation der erforderlichen Elektroden wird ein Siliziumwafer mit einer einseitigen Oxidschicht von ca. 1 µm Dicke auf der oxidierten Seite mit einer Haftschicht, bspw. 3 nm Ti, und einer Goldschicht mit 50-100 nm Dicke durch Sputtern beschichtet. Zur Mikrostrukturierung wird eine Mehrlagenmaskierung eingesetzt, um die Elektrodenspaltbreiten im unteren Nanometerbereich realisieren zu können. Dazu erfolgt eine Beschichtung mit Kohlenstoff (30 nm) und anschließend einer Metallkombination (Ti bzw. NiCr, 10 nm dick). Anschließend wird ein Elektronenstrahlresist (150nm) aufgeschleudert. Die Belichtung wird durch eine Mix-Match Technologie realisiert, bei der die großflächigen Elektroden 2 mittels einer Formstrahl-Elektronenstrahlbelichtung und die feinen, zwischen den Elektroden 2 befindlichen Spalte mit einer Punktstrahl-Elektronenstrahlbelichtungsanlage erzeugt werden. Die Strukturübertragung erfolgt mittels Ionenstrahl-Ätzen (IBE) in die Metallschicht und reaktivem Ionenätzen (RIE) in die Kohlenstoffschicht. Die Struktur wird mittels eines IBE-Prozeß in die Gold- und Haftschicht übertragen. Anschließend wird die Maskierungsschicht in einem O₂-RIE-Prozeß entfernt, und es erfolgt - gleichzeitig eine Oberflächenaktivierung.
   Es werden im nachfolgenden Techniken beschrieben, die auf der Silanisierung der Chipoberfläche beruhen. Durch diese Silanisierung werden die Oberflächen für die Bindung Amino-modifizierter Oligonukleotide aktiviert. Es sollen hier zwei verschiedene Methoden der Silanisierung und anschließender Immobilisierung ausgeführt werden. Neben der Silanisierung sind aber auch andere Möglichkeiten der Oberflächenaktivierung und Immobilisierung möglich.
B.1. Silanisierung mittels 3-Aminopropyltrimethoxysilan APTES: Wie unter A. beispielhaft beschrieben, werden vorstrukturierte Chips mit Gold-Elektroden in einem Ultraschallbad nacheinander in konz. Salpetersäure, Wasserstoffperoxidlösung (30%) und Wasser gereinigt und anschließend bei 80°C für 5 min getrocknet. Danach werden die Chips für 2 min in 1 %iger Silanlösung in 95% Azeton/Wasser inkubiert. Nach 10-maligem Waschen in Azeton für jeweils 5 min werden die Chips bei 110°C getrocknet. Danach werden sie für 2 h in 0,2%iger Phenylendiisothiocyanatlösung in 10% Pyridin/Dimethylformamid inkubiert und mit Methanol und Azeton gewaschen. Die derart aktivierten Chips können über längere Zeit im Exikator bei 4°C gelagert werden.
   Danach erfolgt die Anbindung der amino-modifizierten Oligonukleotide, wozu ein Tropfen der Lösung der Oligonukleotide (2mM in 100mM Natriumcarbonat/-bicarbonatpuffer) auf den Chip aufgetragen wird. Dabei ist durch die parallele Applikation kleiner Tröpfchen verschiedener Oligonukleotide eine Parallelisierung, bspw. bei Einsatz einer Ausführungsform des Affinitätssensors nach Fig. 4, erreichbar. Das Auftragen genannter Tröpfchen kann durch Mikropipetten, Spotter oder andere verfügbare Techniken zum Auftragen kleiner Probenmengen erfolgen. Anschließend werden die Chips bei 37°C in einer Feuchtkammer für 1-2 h inkubiert. Nach Entfernung der Tropfen werden die Chips einmal mit 1% Ammoniaklösung und dreimal mit Wasser gewaschen. Danach erfolgt Trocknung bei Raumtemperatur.
B.2. Eine zweite Möglichkeit der Silanisierung erfolgt mittels 3-Glycidoxypropyltrimethoxysilan (GOPS), wozu die Chips, wie unter B.1. beschrieben, gereinigt und anschließend für jeweils 12 min im Ultraschallbad mit Hexan, Azeton und Ethanol behandelt werden. Danach werden die Chips für 5 min bei 80°C getrocknet. Die Silanisierung erfolgt mit 1 mM GOPS in trockenem Toluol bei 80°C für 6-8 h. Die Chips werden gründlich mit Ethylazetat gewaschen und direkt weiterverwendet.
   Danach erfolgt die Anbindung der amino-modifizierten Oligonukleotide, wozu ein Tropfen einer Oligonukleotidlösung (5-50 µM in 0,1 M KOH) auf den Chip aufgebracht und 6 h bei 37°C in der Feuchtkammer inkubiert wird. Durch die Aufbringung mehrerer Tröpfchen mit verschiedenen Oligonukleotiden ist wiederum eine Parallelisierung, wie unter B.1. genannt, erreichbar. Danach läßt man die Tropfen eintrocknen und wäscht mit Wasser bei 50°C unter permanentem Schütteln, worauf sich eine Trocknung bei Raumtemperatur anschließt.
C. Unter diesem Abschnitt soll eine Möglichkeit der Markierung von "probe"-Oligonukleotiden mit kolloidalem Gold beschrieben werden. Dazu bedarf es zunächst einer Vorbereitung der thiolisierten Oligonukleotide, die wie folgt vornehmbar ist: Die mit 3'-Alkylthiol modifizierten Oligonukleotide sind herstellerseitig mit einer Dithiol-Verbindung festphasengebunden, um ihre Funktionsgruppen zu schützen. Durch Abspaltung vom Trägermaterial wird die Funktionsgruppe freigegeben und befindet sich im aktiven Zustand. Die Abtrennung erfolgt in 50 mM DTT (Dithiothreitol) in konzentriertem Ammoniumhydroxid bei 55°C für 16 h (Ansatz: 4-8 mg festphasengebundenes Oligonukleotid, 450 µl Wasser, 50 µl 1M DTT, 50 µl cc Ammoniumhydroxid). Nach der Inkubation wird die flüssige Phase von der Festphase CPG getrennt und vermittels einer Säulenchromatographie entsalzt. Die Oligonukleotide werden in Reaktionspuffer eluiert. Die Konzentration der einzelnen Chromatographie-Fraktionen wird spektralphotometrisch ermittelt.

Der Reaktionsansatz wird bei 55°C für 16 h bei 600 Umdrehungen/min in einem Thermomixer inkubiert, und anschließend für 2-3 min bei einer Beschleunigung von ca. 16000 m/s² zentrifugiert. Derart vorbereitete Fraktionen sind bei -20°C über 4 Wochen lagerbar.
Die Bindung der thiolisierten Oligonukleotide an kolloidales Gold soll beispielhaft wie folgt beschrieben werden:
Zu 5 ml Goldlösung (ca. 17 nM) werden 2,5 OD (260 nm) [OD (260 nm): optische Dichte bei 260 nm] Alkylthiol-Oligonukleotide gegeben (Endkonzentration 3,6 nM). Nach einer Vorinkubation von 16 h bei Raumtemperatur wird nach Einstellung auf 0,1 M NaCl/10 mM Natriumphosphatpuffer, (pH 7,0) 40 h bei Raumtemperatur inkubiert. Anschließend wird für 25 min bei einer Beschleunigung von ca. 16000 m/s² zentrifugiert. Das erhaltene Pellet wird mit 5 ml 0,1 M NaCl/10 mM Natriumphosphatpuffer (pH 7,0) gewaschen. Danach wird erneut für 25 min bei einer Beschleunigung von ca. 16000 m/s² zentrifugiert. Die Redispersion erfolgt in 5 ml 0,3 M NaCl/10 mM Natriumphosphatpuffer (pH 7,0).

Von der so erhaltenen wässrigen Lösung mit kolloidalen Goldpartikeln (Durchmesser im Beispiel 30 nm) werden ca. 40 µl in den Bereich 4 zwischen den Elektroden 2 appliziert. Nach Troclamng zeigen weiter oben beschriebene elektrische Messungen, daß die Strom-Spannungs-Kennlinie einen linearen Verlauf aufweist, was auf ein Ohmsches Verhalten der aggregierten Goldkolloide im betrachteten Bereich hinweist. Bei einer an den Elektroden 2 anliegenden Spannung von ca. 0,3 V konnte ein Strom von 0,3 µA gemessen werden.

Der Affinitätssensor, bspw. in Form des in den Figuren 3 und 4 dargestellten Affinitätschips, kann vielseitig Verwendung finden, so z.B. in der Molekularbiologie und medizinische Diagnostik, wo spezifische Bindungen von bioaktiven Molekülen an ihre korrespondierenden Bindungspartner, bspw. DNA, zu bestimmen sind.
Mit dem Affinitätssensor ist es auf der Grundlage des elektrischen Nachweises spezifischer molekularer Bindungsereignisse in schneller, empfindlicher und spezifischer Weise möglich, ein Biomonitoring, z.B. von Molekülen, Viren, Bakterien und Zellen in verschiedensten Proben, bspw. in klinischen Proben, in Nahrungsmittel- und Umweltproben, wie z.B. aus Kläranlagen, durchzuführen

### Bezugszeichenliste

- 1 -: Trägersubstrat
- 2 -: Elektroden
- 21 -: Mikroelektroden
- 22 -: Kammelektroden
- 23 -: Schnittstellen
- 24 -: Isolationsschicht
- 3 -: Meß- und Auswerteeinheit
- 4 -: Bereich
- 41 -: Affinitätsfläche
- 42 -: Chipfläche
- 43 -: Referenzfläche
- 5 -: spezifische Bindungspartner
- 51 -: inaktive Bindungspartner
- 6 -: komplementär zugehörige Bindungspartner
- 62 -: elektrisch leitfähige Partikel
- 7 -: spezifische Bindemoleküle
- 8 -: Verstärkerschaltkreis
- 9 -: Mikrochip
- A -: Schnittebene
- b -: Breite des Bereichs 4

## Patentansprüche

1. Affinitätssensor für den Nachweis spezifischer Bindungsereignisse bestehend aus einem Trägersubstrat (I), das mit wenigstens zwei beabstandeten Elektroden (2) versehen ist, die beidseitig einen Bereich (4) erfassen, wobei zumindest dieser Bereich (4) mit immobilisierten spezifischen Bindungspartnern (5) versehen ist, wobei die Bindungspartner Nukleinsäuren sind, die befähigt sind, komplementär zugehörige Bindungspartner (6) direkt oder über weitere spezifische Bindemoleküle (7) zu koppeln und der Bereich (4) mit einer Mindestbreite b so festgelegt ist, daß zumindest ein komplementär zugehöriger Bindungspartner (6), der mit einem elektrisch leitfähigem Partikel (62) versehen ist, in genannten Bereich derart aufnehmbar ist, daß zwischen dem Partikol (62) und den Elektroden (2) jeweils die Möglichkeit zur Ausbildung eines Tunnelkontaktübergangs gewährleistet ist.

2. Affinitätssensor für den Nachweis spezifischer molekularer Bindungsereignisse nach Anspruch 1 **dadurch gekennzeichnet, daß** dem Bereich (4) eine Breite b unterhalb von 800 nm gegeben ist.

3. Affinitätssensor für den Nachweis spezifischer molekularer Bindungsereignisse nach Anspruch 1 **dadurch gekennzeichnet, daß** die immobilisierten spezifischen Bindungspartner (5) auch die Elektroden (2) mit einer, einen Tunneleffekt zulassenden Dicke überdecken.

4. Affinitätssensor für den Nachweis spezifischer molekularer Bindungsereignisse nach Anspruch 1 **dadurch gekennzeichnet, daß** die Elektroden (2) als jeweils zwei paarweise angeordnete Mikroelektroden (21) ausgebildet und mit einem Verstärkerschaltkreis (8) verbunden sind, dem eine Meß- und Auswerteeinheit (3)
zugeordnet ist, so daß ein elektrischer Stromfluß über den Bereich (4) bei an den Elektroden (2) angelegter Spannung detektierbar ist.

5. Affinitätssensor für den Nachweis spezifischer molekularer Bindungsereignisse nach den Ansprüchen 1 und 4 **dadurch gekennzeichnet, daß** die Elektroden (2) herausgeführter Bestandteil des Verstärkerschaltkreises (8) sind.

6. Affinitätssensor für den Nachweis spezifischer molekularer Bindungsereignisse nach den Ansprüchen 1 und 5 **dadurch gekennzeichnet, daß** der Verstärkerschaltkreis (8) Bestandteil eines Mikrochips (9) ist.

7. Affinitätssensor für den Nachweis spezifischer molekularer Bindungsereignisse nach Anspruch 1 **dadurch gekennzeichnet, daß** die Elektroden (2) als kammartig ineinandergreifende Strukturenausgebildet sind, wobei sich zumindest zwischen den jeweils gegenüberliegenden Kammelektroden (22) Affinitätsflächen (41) befinden.

8. Affinitätssensor für den Nachweis spezifischer molekularer Bindungsereignisse nach den Ansprüchen 1 und 7 **dadurch gekennzeichnet, daß** die Kammelektroden (22) und die Affinitätsflächen (41) auf einer gemeinsamen Chipfläche (42) angeordnet sind.

9. Affinitätssensor für den Nachweis spezifischer molekularer Bindungsereignisse nach den Ansprüchen 1 und 8 **dadurch gekennzeichnet, daß** die Chipfläche (42) durch ein Siliziumwafer gebildet ist.

10. Affinitätssensor für den Nachweis spezifischer molekularer Bindungsereignisse nach den Ansprüchen 1 und 8 **dadurch gekennzeichnet, daß** die Chipfläche (42) durch ein Glastarget gebildet ist.

11. Affinitätssensor für den Nachweis spezifischer molekularer Bindungsereignisse nach den Ansprüchen 1 und 7 **dadurch gekennzeichnet, daß** die Kam melektroden (22) geometrisch symmetrisch zu interdigitalen Strukturen und eine Vielzahl der Affinitätsflächen (41) zu einer Matrix angeordnet sind, wobei die außerhalb der Affinitätsflächen (41) verlaufenden Elektroden (2) an ihren Schnittstellen (23) voneinander durch eine zwischen diesen befindliche Isolationsschicht (24) getrennt sind.

12. Affinitätssonsor für den Nachweis spezifischer molekularer Bindungsereignisse nach den Ansprüchen 1 oder 7 **dadurch gekennzeichnet, daß** die Länge der Mikroelektroden (21) 0,1 mm, die Breite b des Bereichs (4) 0,1 µm und seine Wirkhöhe 0,02 µm betragen sowie die Affinitätsfläche (41) zur Chipfläche (42) in einem Verhältnis von 1:10 steht.

13. Affinitätssensor für den Nachweis spezifischer molekularer Bindungseroignisse nach den Ansprüchen 1 oder 7 **dadurch gekennzeichnet, daß** neben den Affinitätsflächen (41) wenigstens eine Referenzfläche (43) vorgesehen ist, die anstelle der spezifischen Bindungspartner (5) inaktive Bindungspartner (51) für Reforenzmessungen tragen.

14. Affinitätssensor für den Nachweis spezifischer molekularer Bindungsereignisse nach Anspruch 1 oder 7 **dadurch gekennzeichnet, daß** die einzelnen Affinitätsflächen (41) in unterschiedlicher Dichte mit den spezifischen Bindungspartnern (5) besetzt sind.

15. Affinitätssensor für den Nachweis spezifischer molekularer Bindungsereignisse nach Anspruch 1 oder 7 **dadurch gekennzeichnet, daß** die einzelnen Affinitätsflächen (41) unterschiedliche spezifische Bindungspartner (5) tragen.

16. Affinitätssensor für den Nachweis spezifischer molekularer Bindungsereignisse nach den Ansprüchen 1, 13, 14 oder 15 **dadurch gekennzeichnet, daß** mehrere Referenzflächen (43) vorgesehen sind, die mit unterschiedlichen inaktiven Bindungspartnern (51) besetzt sind.

17. Affinitätssensor für den Nachweis spezifischer molekularer Bindungsereignisse nach Anspruch 1 **dadurch gekennzeichnet, daß** die spezifischen Bindungspartner (5) koordinative Verbindungen eingehen.

18. Affnitätssensor für den Nachweis spezifischer molekularer Bindungsereignisse nach Anspruch 1 **dadurch gekennzeichnet, daß** die leitfähigen Partikel (62) in einer Größe von 0,1 bis 5 µm festgelegt sind.

19. Affinitätssensor für den Nachweis spezifischer molekularer Bindungsereignisse nach Anspruch 1 **dadurch gekennzeichnet, daß** die leitfähigen Partikel (62) in einer Größe im Nanometerbereich festgelegt sind.

20. Affinitätssensor für den Nachweis spezifischer molekularer Bindungsereignisse nach Anspruch 1 **dadurch gekennzeichnet, daß** die leitfähigen Partikel (62) aus Metallclusterverbindungen bestehen.

21. In-vitro Verwendung des Affinitätssensors für den Nachweis spezifischer molekularer Bindungsereignisse nach einem der vorstehenden Ansprüche **dadurch gekennzeichnet, daß** der Affinitätssensor für die Detektion von komplementär zugehörigen Bindungspartnern (6) in Form von Komplexverbindungen eingesetzt wird.

22. Verwendung des Affinitätssensors für den Nachweis spezifischer molekularer Bindungsereignisse nach Anspruch 21 **dadurch gekennzeichnet, daß** der Affinitätssensor für die Detektion von komplementär zugehörigen Bindungspartnern (6) im Form von Nukleinsäuren eingesetzt wird

23. Verwendung des Affinitätssensors für den Nachweis spezifischer molekularer Bindungsereignisse nach einem der vorstehenden Ansprüche 1 bis 20, **dadurch gekennzeichnet, daß** der Affinitätssensor zum Biomonitoring eingesetzt wird.

24. Verwendung des Affinitätssensors für den Nachweis spezifischer molekularer Bindungsereignisse nach Anspruch 23 **dadurch gekennzeichnet, daß** der Affinitätssensor zur Detektion von Zellen eingesetzt wird.

25. Verwendung des Affinitätssensors für den Nachweis spezifischer molekularer Bindungsereignisse nach Anspruch 23 **dadurch gekennzeichnet, daß** der Affinitätssensor zur Detektion von Mikroorganismen eingesetzt wird.

26. Verwendung des Affinitätssensors für den Nachweis spezifischer molekularer Bindungsereignisse nach Anspruch 23 **dadurch gekennzeichnet, daß** der Affinitätssensor zur Detektion von genetischen und mikrobiellen Erkrankungen eingesetzt wird.

27. Verwendung des Affinitätssensors für den Nachweis spezifischer molekularer Bindungsereignisse nach Anspruch 23 **dadurch gekennzeichnet, daß** der Affinitätssensor zur Detektion der Genexpression eingesetzt wird.

28. Verwendung des Affinitätssensors für den Nachweis spezifischer molekularer Bindungsereignisse nach Anspruch 25 **dadurch gekennzeichnet, daß** der Affinitätssensor zur Detektion von Mikroorganismen in ökologischen Populationen eingesetzt wird.

29. Verwendung des Affinitätssensors für den Nachweis spezifischer molekularer Bindungsereignisse nach Anspruch 23 **dadurch gekennzeichnet, daß** der Affinitätssensor zur modizinischen Diagnostik eingesetzt wird.

## Claims

1. An affinity sensor for detecting specific binding events, consisting of a carrier substrate (I) provided with at least two spaced-apart electrodes (2) which cover an area (4) on both sides thereof, at least said area (4) being provided with immobilized specific binding partners (5), wherein said binding partners are nucleic acids capable of coupling complementarily related binding partners (6) directly or through further specific binding molecules (7), and said area (4) has a minimum width b in such a way that at least one of said complementarily related binding partners (6), which is provided with an electrically conductive particle (62), can be taken up within said area in such a way that the possibility of forming a tunnel contact transition is respectively ensured between said particle (62) and said electrodes (2).

2. The affinity sensor for detecting specific molecular binding events according to claim 1, **characterized in that** said area (4) is given a width b of below 800 nm.

3. The affinity sensor for detecting specific molecular binding events according to claim 1, **characterized in that** said immobilized specific binding partners (5) also cover said electrodes (2) at a thickness which enables a tunnel effect.

4. The affinity sensor for detecting specific molecular binding events according to claim 1, **characterized in that** said electrodes (2) are designed as respectively two microelectrodes (21) arranged in pairs, and connected with an amplifier circuit (8) having assigned a measuring and evaluating unit (3), so that an electric current flow can be detected through area (4) when a voltage is applied to the electrodes (2).

5. The affinity sensor for detecting specific molecular binding events according to claims 1 and 4, **characterized in that** said electrodes (2) are projecting parts of said amplifier circuit (8).

6. The affinity sensor for detecting specific molecular binding events according to claims 1 and 5, **characterized in that** said amplifier circuit (8) is part of a microchip (9).

7. The affinity sensor for detecting specific molecular binding events according to claim 1, **characterized in that** said electrodes (2) are designed as intermeshing structures, wherein affinity areas (41) are provided at least between the intermeshing electrodes (22) respectively facing each other.

8. The affinity sensor for detecting specific molecular binding events according to claims 1 and 7, **characterized in that** said intermeshing electrodes (22) and said affinity areas (41) are arranged on a common chip surface (42).

9. The affinity sensor for detecting specific molecular binding events according to claims 1 and 8, **characterized in that** said chip surface (42) is formed by a silicon wafer.

10. The affinity sensor for detecting specific molecular binding events according to claims 1 and 8, **characterized in that** said chip surface (42) is formed by a glass target.

11. The affinity sensor for detecting specific molecular binding events according to claims 1 and 7, **characterized in that** said intermeshing electrodes (22) are arranged with geometric symmetry to form interdigital structures, and a plurality of said affinity areas (41) are arranged to form a matrix, wherein those electrodes (2) which extend outside said affinity areas (41) are separated from each other at their intersection points (23) by an insulation layer (24) provided between.

12. The affinity sensor for detecting specific molecular binding events according to claims 1 or 7, **characterized in that** the length of said microelectrodes (21) is 0.1 mm, the width b of area (4) is 0.1 µm, and its effective height is 0.02 µm, and the ratio of the affinity area (41) to the surface area of the chip surface (42) is 1:10.

13. The affinity sensor for detecting specific molecular binding events according to claims 1 or 7, **characterized in that**, in addition to said affinity areas (41), at least one reference area (43) is provided which bear inactive binding partners (51) instead of specific binding partners (5) for reference measurements.

14. The affinity sensor for detecting specific molecular binding events according to claims 1 or 7, **characterized in that** the individual affinity areas (41) are occupied by said specific binding partners (5) at different densities.

15. The affinity sensor for detecting specific molecular binding events according to claims 1 or 7, **characterized in that** the individual affinity areas (41) bear different kinds of specific binding partners (5).

16. The affinity sensor for detecting specific molecular binding events according to claims 1, 13, 14 or 15, **characterized in that** several reference areas (43) are provided which are occupied by different kinds of inactive binding partners (51).

17. The affinity sensor for detecting specific molecular binding events according to claim 1, **characterized in that** said specific binding partners (5) undergo coordinative bonding.

18. The affinity sensor for detecting specific molecular binding events according to claim 1, **characterized in that** said conductive particles (62) have sizes of from 0.1 to 5 µm.

19. The affinity sensor for detecting specific molecular binding events according to claim 1, **characterized in that** said conductive particles (62) have sizes within the nanometer range.

20. The affinity sensor for detecting specific molecular binding events according to claim 1, **characterized in that** said conductive particles (62) consist of metal cluster compounds.

21. In-vitro use of the affinity sensor for detecting specific molecular binding events according to any of the preceding claims, **characterized in that** said affinity sensor is employed for the detection of complementarily related binding partners (6) in the form of complex compounds.

22. The use of the affinity sensor for detecting specific molecular binding events according to claim 21, **characterized in that** said affinity sensor is employed for the detection of complementarily related binding partners (6) in the form of nucleic acids.

23. Use of the affinity sensor for detecting specific molecular binding events according to any of the preceding claims 1 to 20, **characterized in that** said affinity sensor is employed for biomonitoring.

24. The use of the affinity sensor for detecting specific molecular binding events according to claim 23, **characterized in that** said affinity sensor is employed for the detection of cells.

25. The use of the affinity sensor for detecting specific molecular binding events according to claim 23, **characterized in that** said affinity sensor is employed for the detection of microorganisms.

26. The use of the affinity sensor for detecting specific molecular binding events according to claim 23, **characterized in that** said affinity sensor is employed for the detection of genetic and microbial diseases.

27. The use of the affinity sensor for detecting specific molecular binding events according to claim 23, **characterized in that** said affinity sensor is employed for the detection of gene expression.

28. The use of the affinity sensor for detecting specific molecular binding events according to claim 25, **characterized in that** said affinity sensor is employed for the detection of microorganisms in ecological populations.

29. The use of the affinity sensor for detecting specific molecular binding events according to claim 23, **characterized in that** said affinity sensor is employed for medical diagnostics.

## Revendications

1. Détecteur d'affinité pour la détection d'événements de liaison spécifiques consistant en un substrat de support (I) qui est muni d'au moins deux électrodes (2) séparées, qui entourent des deux côtés un domaine (4), où au moins ce domaine (4) est muni de partenaires de liaison spécifiques (5) immobilisés, où les partenaires de liaison sont des addes nucléiques qui sont capables de se coupler à des partenaires de liaison (6) associés de manière complémentaire, directement ou par le biais d'autres molécules de liaison spécifique (7), et le domaine (4) est établi avec une largeur minimale b de telle sorte qu'au moins un partenaire de liaison (6) associé de manière complémentaire, qui est muni d'une particule électriquement conductrice (62), peut être reçu dans le domaine cité de telle manière que la possibilité de formation d'un passage par contact tunnel est garantie entre la particule (62) et les électrodes (2).

2. Détecteur d'affinité pour la détection d'événements de liaison moléculaires spécifiques selon la revendication 1 **caractérisé en ce qu'**une largeur b inférieure à 800 nm est attribuée au domaine (4).

3. Détecteur d'affinité pour la détection d'événements de liaison moléculaires spécifiques selon la revendication 1 **caractérisé en ce que** les partenaires de liaison spécifiques immobilisés (5) recouvrent aussi les électrodes (2) en une épaisseur autorisant un effet tunnel.

4. Détecteur d'affinité pour la détection d'événements de liaison moléculaires spécifiques selon la revendication 1 **caractérisé en ce que** les électrodes (2) sont agencées dans chaque cas sous forme de deux microélectrodes (21) disposées par paire et sont reliées à un circuit amplificateur (8) auquel est associé un dispositif de mesure et de traitement (3), de sorte qu'une circulation de courant électrique sur le domaine (4) est détectable quand une tension est appliquée aux électrodes (2).

5. Détecteur d'affinité pour la détection d'événements de liaison moléculaires spécifiques selon les revendications 1 et 4 **caractérisé en ce que** les électrodes (2) sont un constituant en relief du circuit amplificateur (8).

6. Détecteur d'affinité pour la détection d'événements de liaison moléculaires spécifiques selon les revendications 1 et 5 **caractérisé en ce que** le circuit amplificateur (8) fait partie d'une micropuce (9).

7. Détecteur d'affinité pour la détection d'événements de liaison moléculaires spécifiques selon la revendication 1 **caractérisé en ce que** les électrodes (2) sont sous forme de structures qui s'interpénètrent à la manière de peignes, où des surfaces d'affinité (41) se trouvent au moins entre les électrodes en peigne (22) opposées dans chaque cas.

8. Détecteur d'affinité pour la détection d'événements de liaison moléculaires spécifiques selon les revendications 1 et 7 **caractérisé en ce que** les électrodes en peigne (22) et les surfaces d'affinité (41) sont disposées sur une surface de puce commune (42).

9. Détecteur d'affinité pour la détection d'événements de liaison moléculaires spécifiques selon les revendications 1 et 8 **caractérisé en ce que** la surface de puce (42) est formée par une plaquette de silicium.

10. Détecteur d'affinité pour la détection d'événements de liaison moléculaires spécifiques selon les revendications 1 et 8 **caractérisé en ce que** la surface de puce (42) est formée par une cible en verre.

11. Détecteur d'affinité pour la détection d'événements de liaison moléculaires spécifiques selon les revendications 1 et 7 **caractérisé en ce que** les électrodes en peigne (22) sont disposées géométriquement symétriquement en structures interdigitées et une pluralité de surfaces d'affinité (41) sont agencées en une matrice, où les électrodes (2) qui s'étendent à l'extérieur des surfaces d'affinité (41) sont séparées les unes des autres au niveau de leurs jonctions (23) par une couche d'isolation (24) située entre celles-ci.

12. Détecteur d'affinité pour la détection d'événements de liaison moléculaires spécifiques selon les revendications 1 ou 7 **caractérisé en ce que** la longueur des microélectrodes (21) est 0,1 mm, la largeur b du domaine (4) est 0,1 µm et sa hauteur efficace est 0,02 µm, et la surface d'affinité (41) et la surface de puce (42) sont dans un rapport de 1:10.

13. Détecteur d'affinité pour la détection d'événements de liaison moléculaires spécifiques selon les revendications 1 ou 7 **caractérisé en ce qu'**il est prévu à côté des surfaces d'affinité (41) au moins une surface de référence (43) qui, à la place des partenaires de liaison spécifiques (5), porte des partenaires de liaison inactifs (51) pour des mesures de référence.

14. Détecteur d'affinité pour la détection d'événements de liaison moléculaires spécifiques selon la revendication 1 ou 7 **caractérisé en ce que** les différentes surfaces d'affinité (41) sont occupées par les partenaires de liaison spécifiques (5) en une densité variable.

15. Détecteur d'affinité pour la détection d'événements de liaison moléculaires spécifiques selon la revendication 1 ou 7 **caractérisé en ce que** les différentes surfaces d'affinité (41) portent des partenaires de liaison spécifiques (5) différents.

16. Détecteur d'affinité pour la détection d'événements de liaison moléculaires spécifiques selon les revendications 1, 13, 14 ou 15 **caractérisé en ce qu'**il est prévu plusieurs surfaces de référence (43) qui sont occupées par des partenaires de liaison inactifs (51) différents.

17. Détecteur d'affinité pour la détection d'événements de liaison moléculaires spécifiques selon la revendication 1 **caractérisé en ce que** les partenaires de liaison spécifiques (5) engagent des liaisons de coordination.

18. Détecteur d'affinité pour la détection d'événements de liaison moléculaires spécifiques selon la revendication 1 **caractérisé en ce que** les particules conductrices (62) sont ajustées à une taille de 0,1 à 5 µm.

19. Détecteur d'affinité pour la détection d'événements de liaison moléculaires spécifiques selon la revendication 1 **caractérisé en ce que** les particules conductrices (62) sont ajustées à une taille dans le domaine des nanomètres.

20. Détecteur d'affinité pour la détection d'événements de liaison moléculaires spécifiques selon la revendication 1 **caractérisé en ce que** les particules conductrices (62) consistent en composés de type clusters métalliques.

21. Utilisation in vitro du détecteur d'affinité pour la détection d'événements de liaison moléculaires spécifiques selon l'une des revendications précédentes, **caractérisée en ce que** le détecteur d'affinité est utilisé pour la détection de partenaires de liaison (6) associés de manière complémentaire sous forme de composés complexes.

22. Utilisation du détecteur d'affinité pour la détection d'événements de liaison moléculaires spécifiques selon la revendication 21 **caractérisée en ce que** le détecteur d'affinité est utilisé pour la détection de partenaires de liaison (6) associés de manière complémentaire sous forme d'addes nucléiques.

23. Utilisation du détecteur d'affinité pour la détection d'événements de liaison moléculaires spécifiques selon l'une des revendications 1 à 20 précédentes **caractérisée en ce que** le détecteur d'affinité est utilisé pour le biomonitoring.

24. Utilisation du détecteur d'affinité pour la détection d'événements de liaison moléculaires spécifiques selon la revendication 23 **caractérisée en ce que** le détecteur d'affinité est utilisé pour la détection de cellules.

25. Utilisation du détecteur d'affinité pour la détection d'événements de liaison moléculaires spécifiques selon la revendication 23 **caractérisée en ce que** le détecteur d'affinité est utilisé pour la détection de micro-organismes.

26. Utilisation du détecteur d'affinité pour la détection d'événements de liaison moléculaires spécifiques selon la revendication 23 **caractérisée en ce que** le détecteur d'affinité est utilisé pour la détection de maladies génétiques et microbiennes.

27. Utilisation du détecteur d'affinité pour la détection d'événements de liaison moléculaires spécifiques selon la revendication 23 **caractérisée en ce que** le détecteur d'affinité est utilisé pour la détection de l'expression génique.

28. Utilisation du détecteur d'affinité pour la détection d'événements de liaison moléculaires spécifiques selon la revendication 25 **caractérisée en ce que** le détecteur d'affinité est utilisé pour la détection de micro-organismes dans des populations écologiques.

29. Utilisation du détecteur d'affinité pour la détection d'événements de liaison moléculaires spécifiques selon la revendication 23 **caractérisée en ce que** le détecteur d'affinité est utilisé pour le diagnostic médical.
